# EUROPEAN PATENT APPLICATION

(11) **EP 4 574 151 A1**
(43) Date of publication of application: **25.06.2025**
(21) Application number: 24218075.0
(22) Date of filing: 06.12.2024
(51) Int. Cl.: A61K 31/473, A61P 35/00

(54) **INHIBITORS OF G12V-MUTATED RAS PROTEIN TO TREAT CANCER**

(30) Priority: 21.12.2023 PL PL
(71) Applicant: Personather Spolka Z Ograniczona Odpowiedzialnoscia, 95-050 Konstantynow Lodzki (PL)
(72) Inventor: Rieske, Piotr, 91-013 Lodz (PL); Stoczynska - Fidelus, Ewelina, 96-100 Skierniewice (PL); Wlodarczyk, Aneta, 95-050 Konstantynow Lodzki (PL)
(74) Representative: Kondrat, Mariusz

(57) **Abstract**

The present invention relates to the treatment of cancer patients characterized by cancer cells exhibiting the presence of a mutated gene encoding the RAS protein. The mutation involves codon twelve of this gene (conversion of glycine (G) to valine (V); G12V) and leads to a misalignment within the active site of the RAS protein, thereby preventing the hydrolysis reaction of GTP to GDP. At present, there are no small-molecule substances or other inhibitors available in the oncology clinic that are specific against G12V mutants of the RAS protein.

An object of the invention is the use of a chemical compound with its derivatives that restores the ability to restore GTP hydrolysis in targeted and personalized therapy in cancer patients diagnosed with mutant RAS protein.

## Description

### Specification of the technical field of the invention

The present invention relates to the use of a chemical compound known as 6-phenyl-5,6,6a,7,8,9,10,10a- octahydro-7,10-methanophenanthridine-2-carboxamide (IUPAC: 10-phenyl-9azatetracyclo [10.2.1.02, 11.03,8] pentadeca-3(8),4,6-triene-5-carboxamide) and its derivatives, as well as a pharmaceutical composition containing this compound as an active substance, in the therapy of patients with cancerous diseases characterized by the presence of cancer cells with mutated RAS proteins. The mutation affects the twelfth codon of the RAS genes and leads to the substitution of glycine with valine. The invention relates to personalized and targeted therapy.

### Description of the technical condition

RAS proteins belong to the family of small GTPases, which function as molecular switches by alternating between inactive and active states. Their activation is regulated by GEF proteins (guanine nucleotide exchange factors) catalyzing the exchange of GDP for GTP. RAS proteins (bound to GTP) can transduce (transmit) signals from growth factor receptors to effector proteins - bind to, for example, Raf, PI3K and RalGDS. Through signal transduction, they can influence cell proliferation, differentiation, and growth. Transient inactivation of the transduction ability of RAS protein occurs with the involvement of the GTPase-activating protein, which increases the GTPase activity of RAS proteins by several orders of magnitude. Therefore, GAP facilitates the hydrolysis of GTP, leading to a transient loss of the ability of RAS protein (bound to GDP as well as after GDP release) to transduce signals activated by growth factor receptors.

In the human genome, three genes encode RAS proteins: HRAS, NRAS, and KRAS. All of them share 90% similarity in the sequence encoding the catalytic domain (Illustration 1), covering residues 1-166, but show differences in the sequence in the C-terminal fragment covering the HVR region (hypervariable region). Post-translational modifications of the HVR region allow RAS protein to attach to the cell membrane. The frequency and distribution of mutations are not the same. KRAS is most frequently mutated (86%), while NRAS is the least frequently mutated (3%). Up to 98% of oncogenic mutations involve residues G12, G13, and Q61, causing disruptions in GTP hydrolysis. In human tumors, the most frequently mutated residue is G12, with the most common mutants being G12D (36%), G12V (23%), and G12C (14%).

The G12V mutation of the RAS protein occurs in many types of cancer - pancreatic cancer, colorectal cancer, bladder cancer, lung cancer, ovarian cancer, melanoma, and leukemia [COSMIC]. Each year, approximately 1 million patients are newly diagnosed with this alteration, which explains the tremendous demand for therapy targeting RASG12V [Stewart 2014].

RAS proteins are very similar. To illustrate some of their functions, one can use the example of one of the isoforms of the KRAS protein - KRAS4B, which is composed of two domains: the catalytic (1-166) and the HVR domain (167-188), which undergoes post-translational modifications, allowing the protein to bind to the membrane.

The catalytic domain consists of 6 β-sheets, surrounded by 5 α-helices and binding loops. The functional loop P (G10-G15) and switch regions I (Y32-D38) and II (A59-M67) serve as an active site for GTP hydrolysis and as a binding site for effector proteins (GAP, Raf, PI3K). The G13, Y32, and Q61 residues are involved in the formation of binding interactions with GTP.

In the active site of the correct KRAS4B (wtKRAS4B) (Illustration 2):
- the rest of G13 participates in the formation of a hydrogen bond with the oxygen atom of βγ GTP (oxygen between the β and γ phosphorus);
- the side chain of Q61 interacts with the catalytic water, which forms a hydrogen bond with the γ phosphorus - the carbonyl group of Q61 extracts a hydrogen atom from the catalytic water, and simultaneously the negative hydroxyl ion can attack the γ phosphate of GTP, leading to its hydrolysis;
- the GAP protein provides an arginine finger (R789), which occupies a place in the active center of KRAS, forming polar interactions with the β and γ phosphates of GTP; its role is to neutralize the negative charge in the transition state;
- the side chains of K16, T35, and G60 stabilize the γ phosphate by forming hydrogen bonds either directly with the oxygen atoms of this phosphate group or by coordinating magnesium ions, which in turn interact with the oxygen atoms of β and γ phosphates (T35).

When GTP is bound to RAS, the functional domains form a closed conformation of the GTP binding site. After GTP is hydrolyzed to GDP, these domains loosen and adopt an open conformation, which allows GDP to dissociate. The most significant differences occur in the switch I and II regions.
- when GTP is bound, the phenyl group Y32 adopts an "up" conformation pointing towards the γ- phosphate of GTP, which undergoes hydrolysis;
- when GDP is bound, the phenyl group Y32 adopts a "downward" conformation, and a side chain Q61 adopts an "outward" conformation at the active site.

The correct GTP-associated KRAS4B exists in two conformations: active and inactive. The active state is stabilized by interactions of T35 and G60 with the γ-phosphate. The inactive state consists of two substates; the first one is described by the loss of interactions of the γ-phosphate group with T35 and G60, and the second one by the loss of interactions only with T35.

### The impact of mutations on RAS-GAP interactions

The G12C mutation significantly disrupts the positioning of R789, which moves away from GTP in the active site. Such an influence is not observed with other mutations - R789 is able to form polar interactions with the βγ oxygen of GTP [Lu 2016]. Mutations that cause disturbances in the conformation of the Q61 side chain include:
- G12C - inability to form a hydrogen bond between NE2 Q61 and γ-phosphate;
- G12V i G12D - displacement of OE1 Q61 so that it cannot interact with water;
- G13D - disruptions in the position of Q61 - lack of direct interactions between the side chain of Q61 and GTP.

In summary, mutations of glycine 12 and 13 cause disturbances in the proper positioning of Q61 in the active site of the RAS protein, thereby preventing the hydrolysis reaction of GTP to GDP. Mutations in codons 12 and 13 also hinder access to the GTPase center of the arginine finger of the GAP protein, which ensures efficient hydrolysis. This significantly increases the time of signal transduction from receptors for growth factors.

### GTP hydrolysis mechanism

Among the critical features of the reaction mechanism, a specific alignment of catalytic water in an almost linear arrangement of three atoms has been identified: O[H2O]-Pγ-Oβγ [Khrenova 2014]. The distance in equilibrium from oxygen [H2O] to gamma phosphorus (Pγ) is 3.22 Å, which is consistent with previous results for enzymatic hydrolysis of GTP and ATP. Proper alignment of water in the reaction complex is due to the network of hydrogen bonds formed by residues of T35 and Q61. All three oxygen atoms from GTP gamma phosphate are tightly bound to surrounding groups (Illustration 3).
- O2 is maintained by the Mg2+ ion
- O3 is involved in forming a hydrogen bond with R789 (GAP)
- O1 forms hydrogen bonds with the protonated amino group of K16 and the amide group of Q61.

*In silico* studies on the RAS protein and the RAS-GAP complex catalyzing the hydrolysis of GTP allowed for the description of the individual chemical steps of the hydrolysis reaction [Khrenova 2015]:
1. cleavage of the γ-phosphate-oxygenβγ bond in GTP with the presence of a properly positioned water molecule;
2. formation of a new bond between the γ-phosphate and the oxygen from water;
3. redistribution of protons between the groups involved in the reaction, including Gln61.

The enzyme-substrate state is the basic state, when the distance between the oxygen from the catalytic water and the γ-phosphate is approximately 3 Å (3.22). When this distance decreases, the system passes through the transition state (TS1) to the intermediate state 1 (IT1) - the bond between the γ-phosphate and the oxygen from the βγ in GTP is broken, but the water molecule is still unchanged, i.e., it forms a hydrogen bond with the carbonyl oxygen Q61 and the γ-phosphate of GTP (Illustration 4). As the distance between the oxygen atom of H2O and the γ-phosphate decreases further, a hydroxyl ion attaches to the phosphate atom, and protons rearrange between water, the γ-phosphate, and the Q61 side chain, resulting in the formation of inorganic phosphate H2PO4-. The distance between the hydrogen atom of the Q61 amino group and the oxygen atom of the resulting inorganic phosphate decreases - a stable bond is formed, and the bond between the hydrogen atom of the water molecule (the oxygen from this water is already bonded to the γ-phosphate) and the oxygen atom of the Q61 carbonyl group lengthens, leading to the formation of a hydrogen bond between these atoms.

The IT2 state refers to a situation where, after hydrolysis, GDP and inorganic phosphate are present in the active site of the protein, but the enzyme itself has not yet been regenerated - the Q61 side chain is in an imine form rather than an amide form.

The regeneration of the enzyme involves several transitional states (Illustration 5).
- the path from state I2 to I3 involves the rotation of the hydroxyl group in inorganic phosphate around a single P-O bond, leading to the formation of a hydrogen bond between the oxygen atom of β-phosphate and the OH group of Pi;
- then, the rotation of another hydroxyl group around another P-O bond in inorganic phosphate occurs (transition from I3 -> I4); the hydrogen bond between K16 and the hydrogen atom is broken, the hydrogen atom is transferred from Pi to the imidazole group Q61, and the proton attached to the oxygen atom in Q61 is transferred to Pi;
- finally, the enzyme-product complex is formed, in which the inorganic phosphate forms hydrogen bonds with the oxygen atom at β-phosphate and with the side chain of regenerated glutamine 61.

### G12V mutation vs. RAS-GTP-GAP complex

The G12V mutation in the RAS protein leads to:
- displacement of the Q61 side chain from GTP (hydrogen bonding with the O1 γ-phosphate does not occur);
- displacement of the catalytic water from its favorable location in the active site;
- the function of the arginine finger of the GAP protein is hindered

These shifts cause changes in distances between important atoms in the active center. Therefore, the change from glycine to valine stops GTP within the hydrolytic domain of the RASG12V protein.

As a result, the time of signal transduction from growth factor receptors by RASG12V is significantly prolonged.

### Search for oncogenic blockers of RAS protein forms

For many years, molecules that would directly bind to RAS proteins and block their oncogenic activity have been sought. However, there are no documented studies on molecules specifically targeting RASG12V mutants. Several compounds that bind to RAS proteins have been described, although few binding pockets on the protein surface have been identified as suitable for high-affinity binding. The GDP/GTP binding pocket itself has been ruled out as a useful binding site because:
□ is identical for all GTPases;
□ the affinity of GTP/GDP to the RAS binding site is expressed in picomoles, and their concentration in the cytoplasm is very high.

The identification of molecules targeting the mutated RASG12C encourages the search for other compounds selectively directed towards the mutated RAS protein.

Drugs that covalently modify reactive cysteines, including dacomitinib and ibrutinib, have already been approved for other cancer indications. However, their use is not possible in the case of the G12V mutation.

Various approaches are used in the search for RAS protein function blockers:
□ blocking the binding of RAS to the cell membrane:
□ farnesyltransferase inhibitors (FTIs) □ farnesyl analogs
□ compounds that modulate the distribution of membrane lipids □ inducing cytoplasmic accumulation of inactive RAS
□ blocking proteins responsible for the localization of RAS in the membrane (e.g., PDEdelta)
□ blocking the activity of protein RAS activators such as GEF blocking downstream effectors

It is already known that most of these strategies have not been successful:
□ it turned out that K- and NRAS can be geranylgeranylated in cells treated with FTI, and therapy with multiple prenylation blockers is toxic;
□ blocking the RAF protein (RAS effector) leads to the activation of the RAS protein instead of its deactivation;
□ a significant portion of these substances is toxic because they affect both active RAS proteins in normal cells and cancer cells in a similar way, making their toxicity very high.

The subject of the presented patent application is a group of compounds that have the potential to be effective against RASG12V positive cells. Currently, there are no small molecules specific for G12V mutant RAS proteins available in oncology clinics, let alone those that reactivate their GTPase function. All the compounds described above were intended to block both normal and mutated RAS proteins. As a result, toxic effects on normal cells that should undergo proliferation after activation of growth factor receptors had to occur. These could include normal stem cells or other rapidly proliferating cells. The proposed solution does not involve blocking the action of the RASG12V protein, but rather restoring at least some of its GTPase activity, which is intended to halt oncogenic signal transduction.

### The essence of the invention

The subject of the invention is the chemical compound 6-phenyl-5,6,6a,7,8,9,10,10a-octahydro-7,10 methanophenanthridine-2-carboxamide (IUPAC: 10-phenyl-9-azatetracyclo[10.2.1.0²,11.0³,8]pentadeca- 3(8),4,6-triene-5-carboxamide) and its derivatives, as well as a pharmaceutical composition containing this compound as the active substance, where R is a group: 2H-1,3-benzodioxole; 2,3-dihydro-1,4- benzodioxane; cyclopentyl; 2-chlorophenyl; 4-chlorophenyl; 4-fluorophenyl; 4-methoxyphenyl; 4-(prop- 2-yl)phenyl; 3,4-difluorophenyl; 3-chloro-4-fluorophenyl; 4-fluorophenyl; 3,4-dimethylphenyl; 3,4- dimethoxyphenyl; 4-bromophenyl; 3,4-difluorophenyl; 3-chloro-4-methylphenyl; 4-ethylphenyl; 4-bromo- 3-chlorophenyl, described by the formula (Formula 1) and its derivatives, and a pharmaceutical composition containing this compound as the active substance, for use in the treatment of cancer in patients whose cancer cells show the presence of mutated RAS protein. The mutation affects the twelfth codon of the gene encoding this protein and leads to the substitution of glycine with valine.

It is advantageous that a cancer stem cell with the RASG12V mutation is prone to both cytotoxic and cytostatic effects. Fortunately, the cell is a human cancer cell.

### Beneficial effects of the invention

Advantageously, the chemical compound described by formula (I) is:
□ minimally toxic at physiological and therapeutic concentrations towards normal cells;
□ non-toxic at physiological and therapeutic concentrations towards normal cells;
□ harmless to normal cells at physiological and therapeutic concentrations;
□ shows slight toxicity compared to small molecule chemical compounds used in oncologic therapy.

Beneficially, the effect of the chemical compound described by formula (I) is:
□ stopping cell division of cancer cells;
□ death of cancer cells

Advantageously, such an effect is observed in RASG12V-positive cells.

Beneficially the cell is a cancer cell, e.g. colorectal cancer.

Favorably, the cell is a tumor cell other than colorectal cancer in which at least some of the cells exhibit the RASG12V mutation.

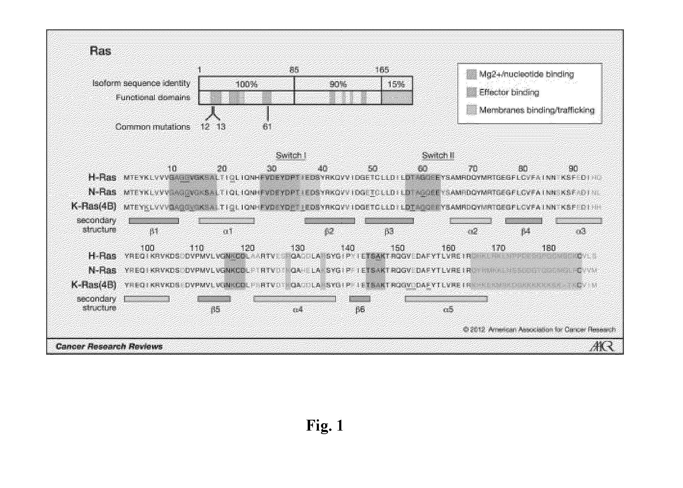

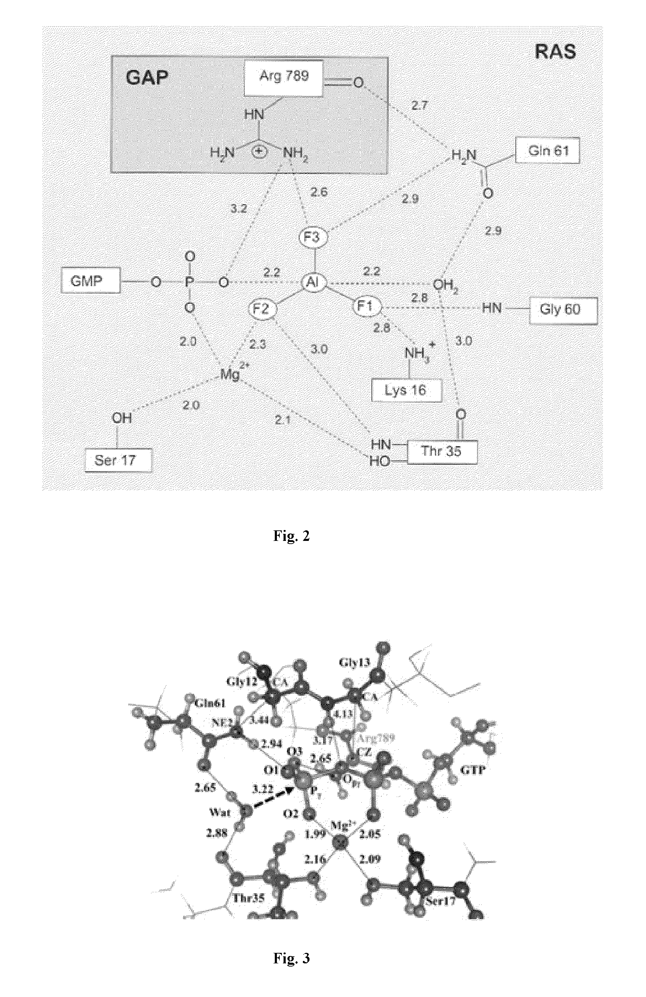

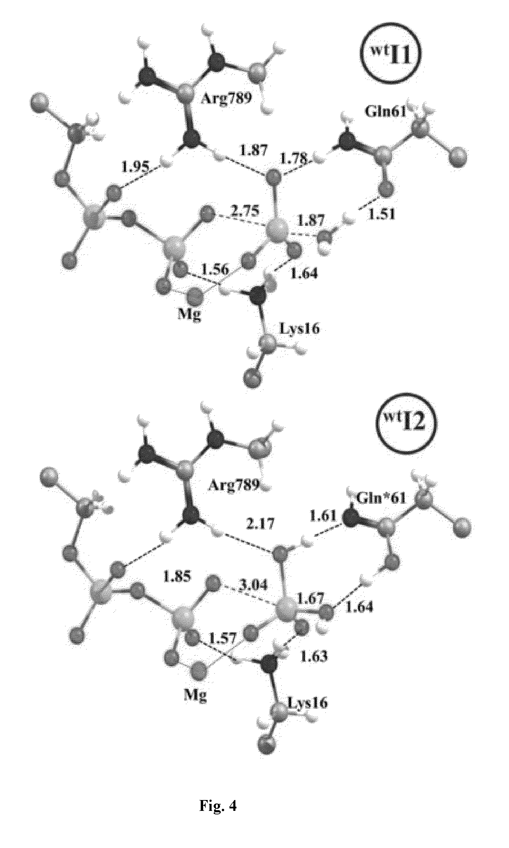

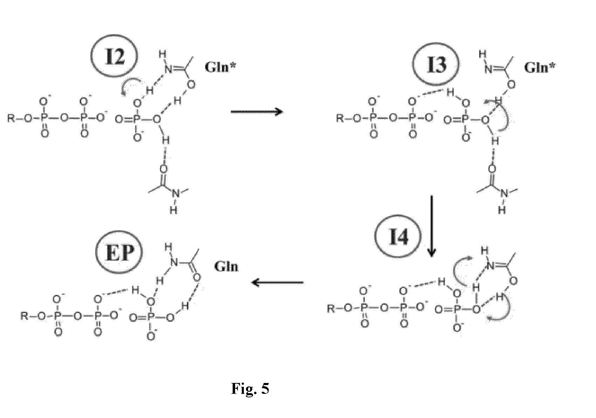

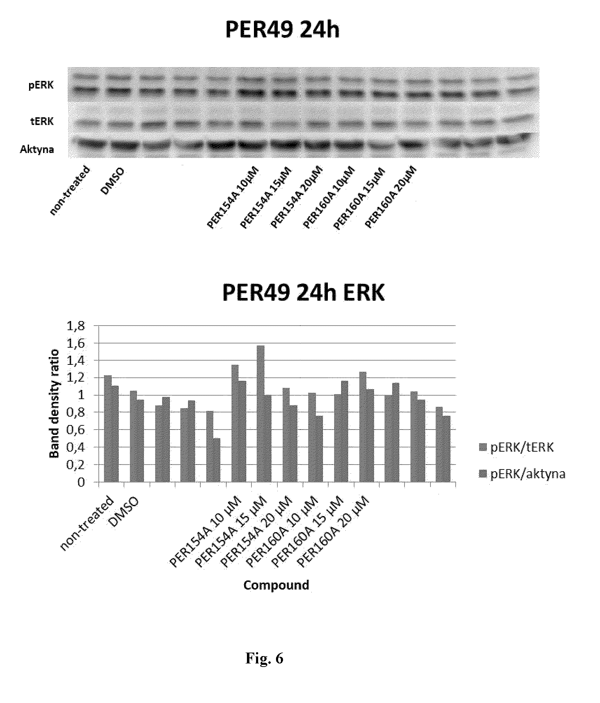

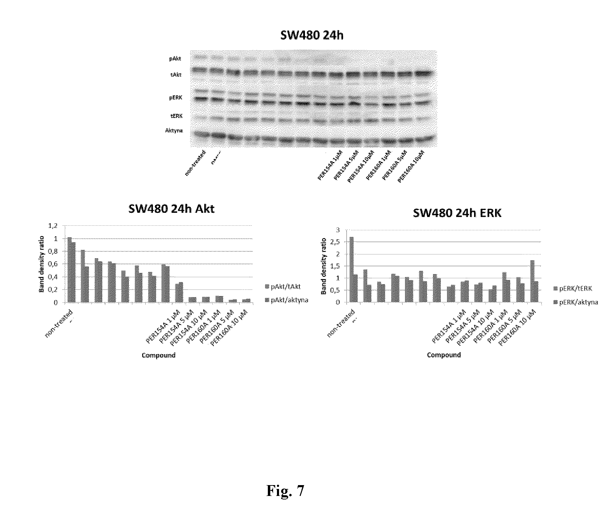

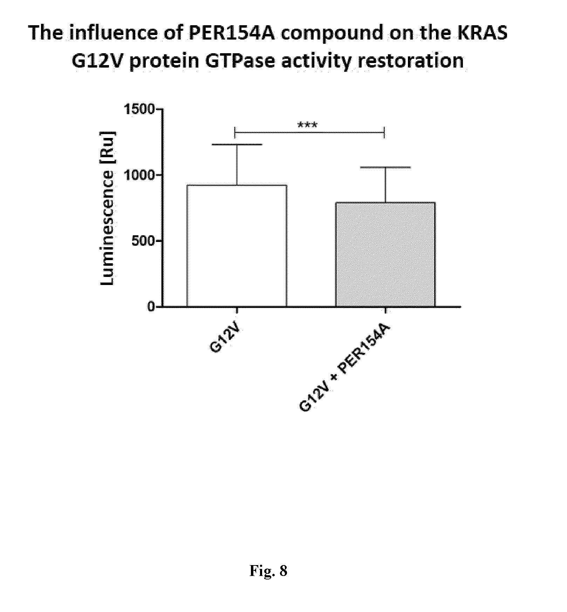

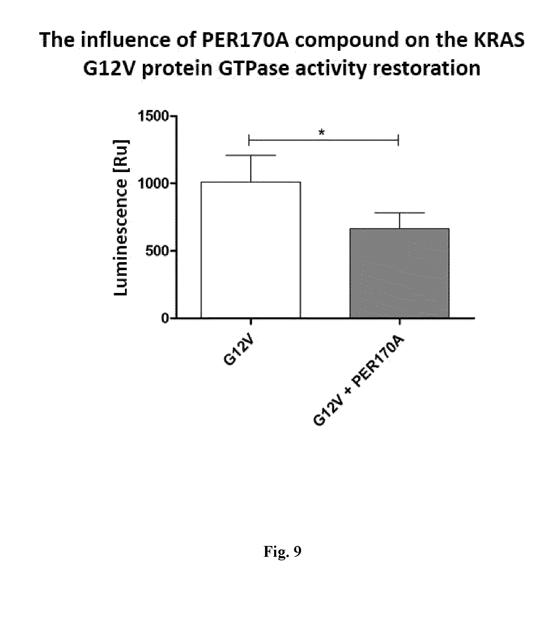

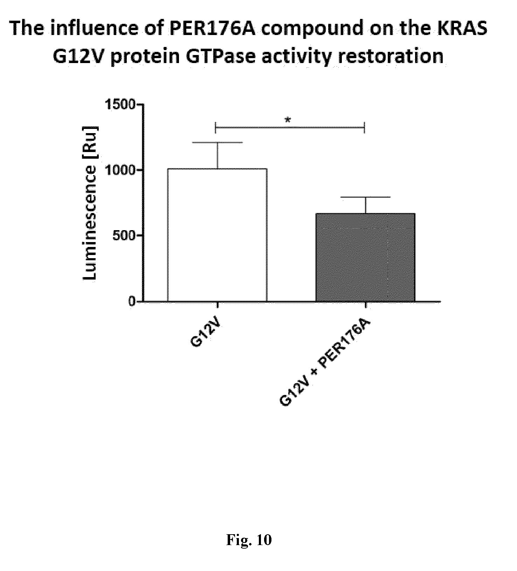

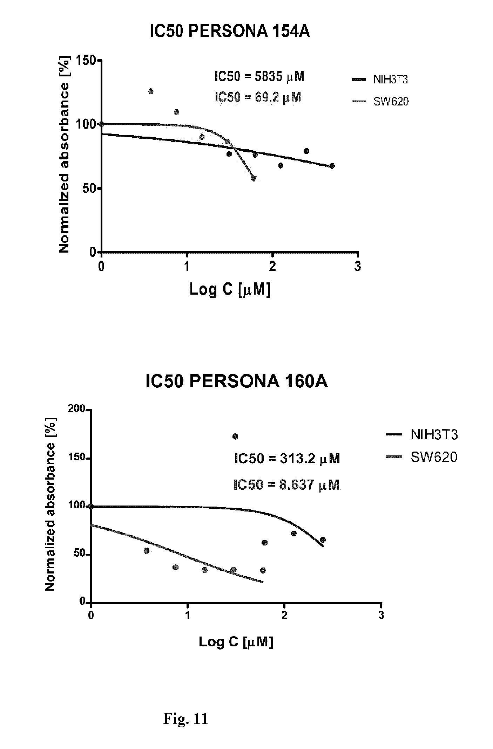

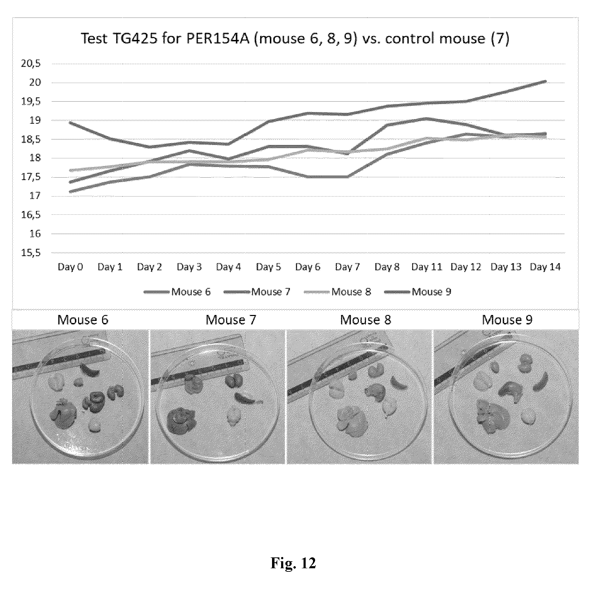

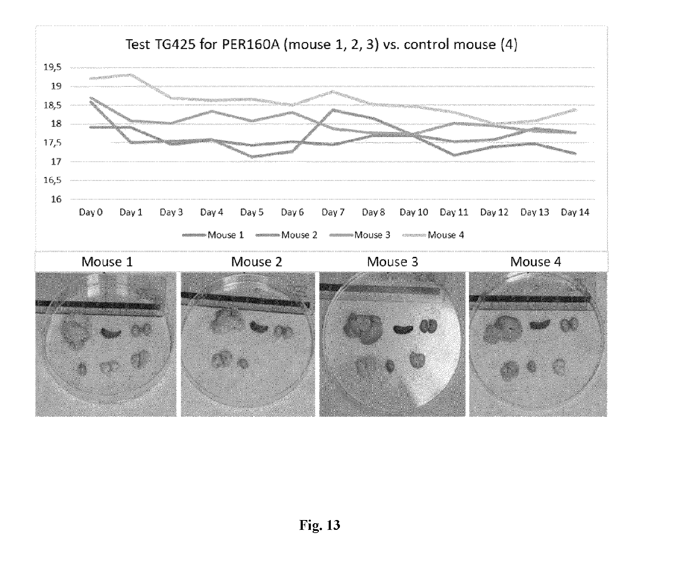

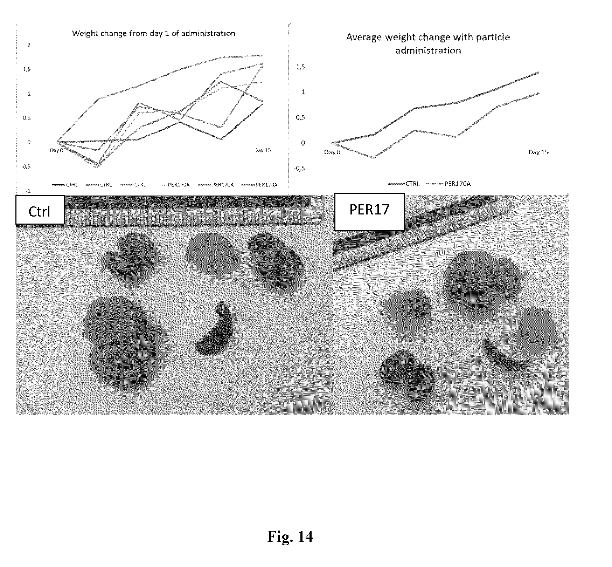

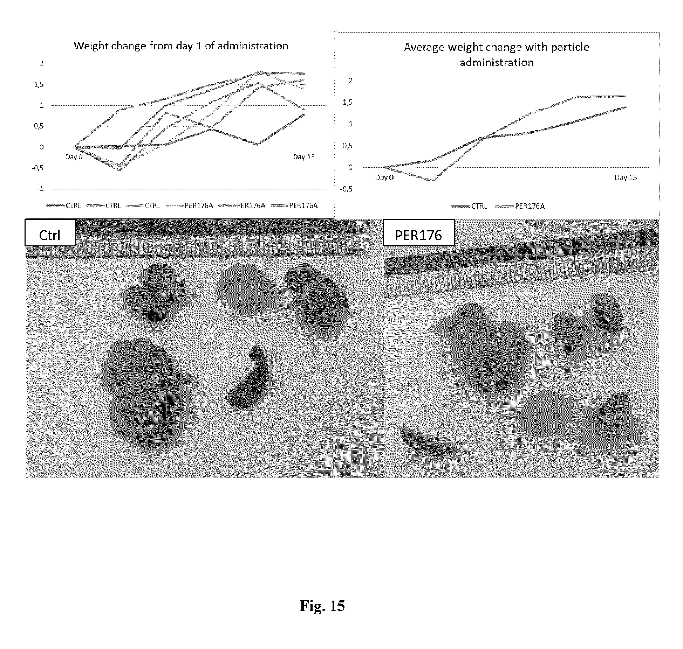

### Explanation of the figures of the drawing

The subject of the invention in examples of implementation is shown in the drawings:
**Illustration 1.** Sequences of catalytic domains of RAS proteins - HRAS, NRAS, and KRAS [Prior 2012].
**Illustration 2.** Interactions between the substrate and the amino acids of the RAS binding center [Scheffzek 1997].
**Illustration 3.** Interactions between the atoms of the residues of the RAS GTP active center [Scheffzek 1997].
**Illustration 4.** The first step of GTP hydrolysis.
**Illustration 5.** Regeneration of the RAS protein after GTP hydrolysis has been carried out.
**Illustration 6.** Western blot analysis for the molecule labeled as PER154A and PER160A. No impact on the signaling transduction pathway in the normal PER49 cell line.
**Illustration 7.** Western blot analysis for the molecule labeled as PER154A and PER160A. Significant impact on the AKT signaling transduction pathway in SW480 cell line with RASG12V mutation.
**Illustration 8.** Measurement of GTPase activity of mutated RASG12V protein after application of PER154A molecule in GTPase-Glo^{™} Assay (Promega).
**Illustration 9.** Measurement of GTP-ase activity of mutated RASG12V protein after application of PER170A molecule in GTPase-Glo^{™} Assay test (Promega).
**Illustration 10.** Measurement of GTPase activity of mutated RASG12V protein after application of PER176A molecule in GTPase-Glo^{™} Assay test (Promega).
**Illustration 11.** Measurement curves of IC₅₀ values based on the crystal violet test for molecules PER154A and PER160A in SW620 cells (with RASG12V mutation) and normal NIH3T3 cells.
**Illustration 12.** Molecule PER154A orally administered to three BALB/c mice (6, 8, and 9) in an acute toxicity test after oral administration (Acute Toxicity test TG425). Mouse number 7 - control mouse; the Y axis represents the weight of the animals. Appearance of internal organs of tested and control mice after euthanasia. The compound PER154A was found to be safe for mice. No visual signs of toxicity.
   Molecule Per160A orally administered to three BALB/c mice (mouse 1, 2, and 3) in an acute toxicity test after oral administration (Acute Toxicity test TG425). Mouse number 4 - control mouse; the Y axis represents the weight of the animals (A). Appearance of internal organs of tested and control mice after euthanasia (B). The compound Per160A was found to be safe for mice. No visual signs of toxicity.
**Illustration 13.** Molecules PER170A and PER176A given orally to three BALB/c mice in the acute toxicity test after oral administration (Acute Toxicity test TG425). In the graphs above, the Y axis represents the change in animal weight from the time of particle administration. The appearance of the internal organs of the tested mice and the control mice after euthanasia (shown in one example photo below the graphs). The compounds PER170A and PER176A proved to be safe for mice. There were no visual signs of toxicity.

### Detailed description of the essence of the invention

The subject of the invention is a chemical compound described by the formula (1) known as 6-phenyl-5,6,6a,7,8,9,10,10a-octa.hydro-7,10-methanophenantrene-2-carboxyamide (IUPAC: 10-phenyl-9 azatetracyclo[10.2.1.02,11.03,8]pentadeca-3(8),4,6-triene-5-carboxamide) and its derivatives, as well as a pharmaceutical composition containing this compound as the active substance for use in anti-cancer therapy in the case of tumors whose cells exhibit expression of the RAS protein with a mutation causing a change from glycine 12 to valine (codon 12 glycine-valine; G12V), where R is a group: 2H-1,3-benzodioxolyl; 2,3-dihydro-1,4-benzodioxanyl; cyclopentyl; 2-chlorophenyl; 4-chlorophenyl; 4-fluorophenyl; 4- methoxyphenyl; 4-(isopropyl)phenyl; 3,4-difluorophenyl; 3-chloro-4-fluorophenyl; 4-fluorophenyl; 3,4- dimethylphenyl; 3,4-dimethoxyphenyl; 4-bromophenyl; 3,4-difluorophenyl; 3-chloro-4-methylphenyl; 4- ethylphenyl; 4-bromo-3-chlorophenyl. The groups (R) in graphic form are included below in the table.

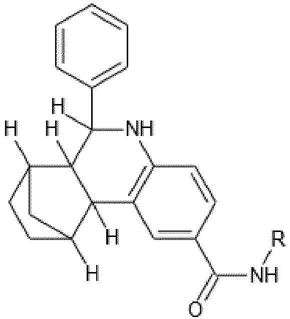

**Formula 1.** The chemical structure of a compound known as 6-phenyl-5,6,6a,7,8,9,10,10a-octahydro-7,10- methanophenanthridin-2-carboxamide (IUPAC: 10-phenyl-9-azatetracyclo[10.2.1.02,11.03,8]pentadeca- 3(8),4,6-triene-5-carboxamide), where R is a group: 2H-1,3-benzodioxolyl; 2,3-dihydro-1,4- benzodioxanyl; cyclopentyl; 2-chlorophenyl; 4-chlorophenyl; 4-fluorophenyl; 4-methoxyphenyl; 4- (propan-2-yl)phenyl; 3,4-difluorophenyl; 3-chloro-4-fluorophenyl; 4-fluorophenyl; 3,4-dimethylphenyl; 3,4-dimethoxyphenyl; 4-bromophenyl; 3,4-difluorophenyl; 3-chloro-4-methylphenyl; 4-ethylphenyl; 4- bromo-3-chlorophenyl.

**Table. Identification of R fragments connected to the core of the molecule, which is the subject of the invention.**

| **Molecule name** | **R (group)** | **R in graphic form** |
|---|---|---|
| PER67A | 2H-1,3-benzodioxole group | 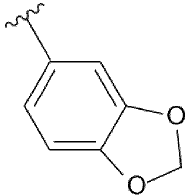 |
| PER161A | | 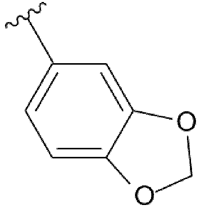 |
| PER154A | 2,3-dihydro-1,4-benzodioxane group | 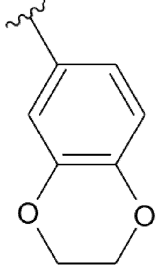 |
| PER155A | cyclopentyl group | 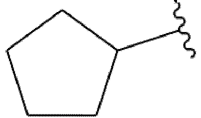 |
| PER156A | 2-chlorophenyl group | 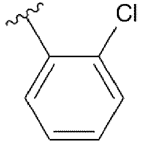 |
| PER159A | 4-chlorophenyl group | 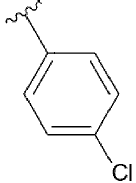 |
| PER166A | Rule As above | 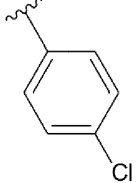 |
| PER160A | 4-fluorophenyl group | 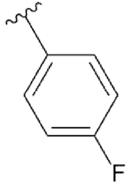 |
| PER162A | 4-methoxyphenyl group | 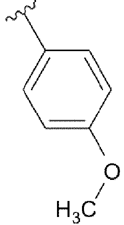 |
| PER163A | 4-(propan-2-yl)phenyl group | 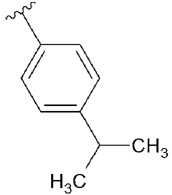 |
| PER165A | 3,4-difluorophenyl group | 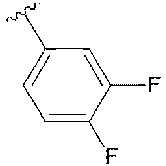 |
| PER168A | 3-chloro-4-fluorophenyl group | 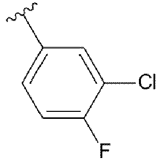 |
| PER169A | 4-fluorophenyl group | 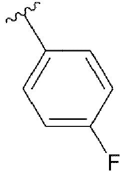 |
| PER170A | 3,4-dimethylphenyl group | 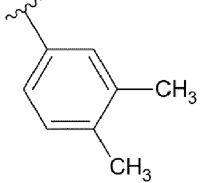 |
| PER171A | 3,4-dimethoxyphenyl group | 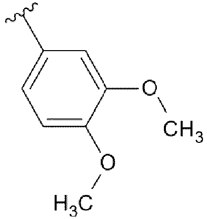 |
| PER172A | 4-bromophenyl group | 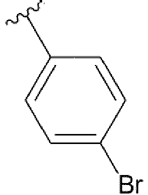 |
| PER173A | 3,4-difluorophenyl group | 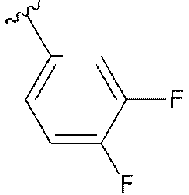 |
| PER174A | 3-chloro-4-methylphenyl group | 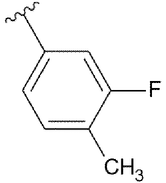 |
| PER175A | 4-ethylphenyl group | 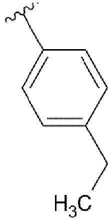 |
| PER176A | 4-bromo-3-chlorophenyl group | 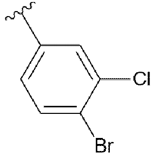 |

The molecules were synthesized and provided, among others, by Molport, Mcule, Specs, or Enamine, and selected from the group of molecules described in the examples were encoded under the names **PER154A, PER160A, PER170A, PER176A.**

| **Name of the molecule from the group** | **R** |
|---|---|
| PER154A | Group 2,3-dihydro-1,4-benzodioxane |
| PER160A | 4-fluorophenyl group |
| PER170A | 3,4-dimethylphenyl group |
| PER176A | 4-bromo-3-chlorophenyl group |

### Molecule as an inhibitor of signal transduction mediated by RASG12V protein

The molecule that is the subject of the invention binds directly to RASG12V and causes a change in the conformation of key amino acids in the active site. Thus, this molecule restores a conformation similar to that known from the RASWT-GAP and RASWT-GAP-GTP complexes. The molecule significantly influences the change in conformation and partially restores the GTPase activity of RASG12V. The source of molecules was the ZINC database. The molecule representing the lead structure was found in a virtual screening process. The screening was conducted by docking molecules to the RASG12V structure (PDB ID: 30IW). The RASG12V-GTP model (in its active state) was built based on the crystallographic structure of the RASG12V protein (PDB code: 30IW). The GNP present in the structure was modified to GTP, and the structure was prepared for docking with standard settings. The compound with the best Glide scoring function value (hit) was verified for estimated physicochemical properties (QikProp), drug-likeness (compliance with Lipinski's rules), synthetic availability (expert opinion), and patentability (patent database review). After verification, the molecule was selected as the lead structure. The lead structure was docked first to the rigid RASG12V protein. To assess the possibility of conformational changes in the RASG12V protein due to binding of the lead structure, docking was performed taking into account the induced fit phenomenon.

The molecules described in the submission come from the ZINC database (zinc15.docking.org) or are the derivatives of them. The leading structure of the molecules was used as a query. The search was conducted in Tanimoto mode - 70. Choosing molecules from ready-made suggestions from manufacturers' catalogs ensures their easy synthetic availability and usually conditions a relatively low purchase price, compared to "virtual" molecules, for example, created by fragment linking or core exchange methods. The docking was conducted in standard mode. As a result of the IFD docking, a maximum of 20 poses were obtained for each analyzed molecule.

In order to correctly assign the measure of RMSD distance from the reference position of key amino acids, it was necessary to modify the original script. Studies conducted in silico before the discovery of the invention described here indicate the possibility of designing a molecule that modulates the functioning of the mutated form of the protein, and in vitro studies directly point to the possibility of using such a molecule in targeted anti-cancer therapy in patients whose cancer cells are characterized by the presence of a mutated RAS protein-coding gene. The mutation affects the twelfth codon of RAS genes and results in the substitution of glycine with valine.

The results of in vitro analyses were presented in the examples. Commercially available cancer cell lines were used for the studies, including SW480, SW620, and COR-L23, showing endogenous expression of KRASG12V, as well as a cell line with exogenously introduced KRASG12V mutation (using methods of genetic engineering) - the DK-MGEGFRvIIIKRASG12V line. Additionally, the cancer cell line NCI- H1975, characterized by the expression of wild type RASWT proteins, was also analyzed.

To evaluate the toxicity of the tested compound on normal cells, the NIH/3T3 cell line - immortalized mouse fibroblasts and normal pericytes (PER49) were used.

In the study, the toxicity of the compound was demonstrated, using molecules PER154A/PER160A/PER170A/PER176A as an example, against cancer cells expressing KRASG12V, while showing no toxicity of this compound against cancer cells expressing normal RAS protein as well as normal cells.

The ability to partially restore the GTPase activity, characteristic of the wild-type RAS protein, to the mutated RASG12V protein was demonstrated after the application of PER154A/PER160A/PER170A/PER176A in a direct test (GTPase activity of RASG12V).

**It should be emphasized that this is a particularly significant result due to the fact that the functional assay is performed on a protein isolated from cancer cells in an extracellular system. This indicates that regardless of the type of cancer in which the mutated RASG12V protein is detected, the molecule will restore normal GTPase activity regardless of clinical diagnosis, and the only condition for its effectiveness is the presence of G12V mutations in RAS proteins** (for example, KRASG12V protein is identical regardless of which cancer cell it is isolated from). It was also shown in phospho WB studies that this molecule leads to a decrease in phosphorylation of AKT and ERK, kinases located downstream (below RAS) in signal transduction pathways from RASG12V. The effects turned out to be statistically significant.

Molecules used as a negative control (erlotinib, deltarasin) did not show any influence on the GTPase activity of RASWT or RASG12V, even at high concentrations, whether in the presence of GAP or in its absence. Importantly, the molecule designed by Personather shows the ability to restore the GTPase activity of RASG12V even in the absence of GAP. Furthermore, the molecules, as expected, allowed for the blocking of protein phosphorylation below the RASG12V protein. These results suggest that molecules designed by Personather increase the GTPase activity of RASG12V to a degree that may serve as the basis for anti-cancer therapy.

The unique features of the molecule for use in therapy and its effectiveness have been demonstrated in the following examples.

### Examples of implementing the invention

### Example 1.

**Analysis of the impact of the studied compound on the signal transduction pathway controlled by RAS family proteins in normal cell lines based on Western Blot analysis.**

The example illustrates the impact of the PER154A and PER160A molecules on the signal transduction pathway in the normal PER49 cell line. To this end, the following study was conducted on the PER49 cell line, which does not exhibit the KRASG12V mutation.

### Day 1

1. PER49 cell line cells at 80-90% confluency were rinsed with phosphate-buffered saline without Mg2+ and Ca2+ ions. Remove PBS.
2. Cells were detached from the vessel using Trypsin-EDTA (0.05%, 1x) for 1-3 minutes at 37°C.
3. Cells were transferred to a 15 ml tube, centrifuged (200xg, 4 minutes) and suspended in RPMI1640 medium with 10% bovine serum and antibiotics.
4. Cells were counted.
5. 3x10⁵cells were added to each well of a 6-well plate for adherent culture.
6. The above medium was added and left for approximately 24 hours.

### Day 2

7. The cells were washed 2-3 times with buffered saline without Mg2+ and Ca2+ ions. Each time PBS was thoroughly removed.
8.2 ml of RPMI1640 medium with antibiotics and without serum was added, and left for approximately 24 hours.

### Day 3

9. The cells were washed 2-3 times with a buffered saline solution without Mg2+ and Ca2+. PBS was thoroughly removed each time.
10. 2 ml of RPMI1640 medium with antibiotics and without serum with the appropriate compounds (DMSO, volumetrically equal to the maximum volume of the tested compounds; PER154A and PER160A, 10, 15, and 20 µM) were added.
11. The cells were incubated with the above compounds for 24 hours.

### Day 4

12. Cells were washed 2-3 times with a buffered saline solution without Mg2+ and Ca2+. PBS was thoroughly removed each time.
13. 80 µl of buffer for phospholysis with protease and phosphatase inhibitors (1:100) was added to each well of a 6-well plate.
14. Cells were detached with a scraper and transferred to Eppendorf tubes.
15. Incubated for 30 minutes on ice with shaking.
16. Spun at 7000 x g, 6 min, 4°C.
17. Supernatant was collected, a portion was left for protein concentration measurement in the sample (20 µl).
18. The remaining 60 µl was suspended in 4X Laemmli Sample Buffer with β-mercaptoethanol, heated to 98°C for 5 min, then stored at -80°C.

### Day 5

19. Polyacrylamide gels were poured (12% separating gel).
20. An electrophoresis apparatus was assembled.
21. 20 µg of protein was loaded into the gel wells.
22. Electrophoretic separation was carried out under conditions of 25 mA, 270 V, const. A, until the samples had separated on the gel.
23. Membranes were activated during the separation by immersing them in methanol for 10 minutes, followed by incubation in transfer buffer for 10 minutes.
24. After electrophoresis was completed, the apparatus was dismantled, Western Blot sandwiches were assembled, placed in a cassette, and transfer was carried out for 75 minutes, 270 V, 270 mA, const. V.
25. The equipment was dismantled, and the membranes were rinsed in TBS-T buffer for 5 minutes at room temperature with shaking.
26. The membranes were then placed in 5% defatted milk or Phospho blocker for 1 hour at room temperature with rocking.
27. The membranes were rinsed 3 times for 10 minutes each in TBS-T buffer.
28. Primary antibodies diluted in TBS-T buffer were added (1:500 mouse anti-ERK 1/2 (C-9) sc-7383, Santa Cruz; 1:500 mouse anti-p-ERK (E-4) sc-7838, Santa Cruz; 1:500 rabbit anti-Akt, 9272S, Cell Signalling; 1:500 rabbit anti-pAkt (S473) (D9E), 4060L, Cell Signalling; 1:4000 mouse anti-Actin, clone C4, MAB1501, Merck) and left overnight at +4°C.

### Day 6

29. The next day, the membranes were brought to room temperature, washed 3 times for 10 minutes with TBS-T buffer.
30. Secondary antibodies diluted in TBS-T buffer were added (goat anti-rabbit IgG HRP, sc-2004, Santa Cruz, 1:4000; m-IgG Kappa-Binding Protein HRP (mouse), sc-516102, Santa Cruz, 1:4000), and incubated for 2 hours at room temperature with shaking.
31. The membranes were washed 3 times for 10 minutes with TBS-T buffer.
32. Visualized using the Amersham ECL Western Blotting Detection reagent kit.

**Results and conclusions:** The molecule labeled as PER154A and PER160A does not show any influence on the signal transduction pathway in normal PER49 cell line (Illustration 6).

### Example 2.

**Analysis of the impact of the tested compound on the signal transduction pathway controlled by RAS family proteins in cancer cell lines expressing KRASG12V based on the Western Blot method.**

The example illustrates the impact of the PER154A and PER160A molecules on the signal transduction pathway in the cancer cell line SW480. The following study was conducted on SW480 cell line, which shows endogenous expression of the KRASG12V mutant:

### Day 1

1. SW480 cell line at 80-90% confluence was rinsed with PBS without Mg2+ and Ca2+ ions. Remove PBS.
2. Cells were detached from the vessel using Trypsin-EDTA (0.05%, 1x) for 1-3 minutes at 37°C.
3. Cells were transferred to a 15 ml tube, centrifuged (200xg, 4 min) and suspended in DMEM High Glucose (HG) medium with 10% bovine serum and antibiotics.
4. Cells were counted.
5. 5x10⁵ cells were added to each well of a 6-well plate for adherent culture.
6. The above medium was replenished and left for approximately 24 hours.

### Day 2

7. Cells were washed 2-3 times with buffered saline solution without Mg2+ and Ca2+ ions. PBS was carefully removed each time.
8. 2 ml of DMEM HG medium with antibiotics and without serum was added, left for approximately 24 hours.

### Day 3

9. The cells were washed 2-3 times with buffered saline without Mg2+ and Ca2+ ions. PBS was carefully removed each time.
10. 2 ml of DMEM HG medium with antibiotics and without serum containing the appropriate compounds (DMSO, volume equal to the maximum volume of tested compounds; PER154A, PER160A 1, 5, and 10 µM) were added.
11. The cells were incubated with the above compounds for 24 hours.

### Day 4

12. Cells were washed 2-3 times with buffered physiological saline solution without Mg2+ and Ca2+ ions. PBS was removed thoroughly each time.
13. 80 µl of a buffer for phospholysis with protease and phosphatase inhibitors (1:100) was added to each well of a 6-well plate.
14. Cells were detached with a scraper and transferred to Eppendorf tubes with the buffer.
15. Incubated on ice with agitation for 30 minutes.
16. Spun at 7000 x g, 6 minutes, 4°C.
17. Supernatant was collected, part of it was saved for protein concentration measurement in the sample (20 µl).
18. The remaining 60 µl was suspended in 4X Laemmli Sample Buffer with β-mercaptoethanol, heated to 98°C for 5 minutes, then stored at -80°C.

### Day 5

19. Polyacrylamide gels (12% separating gel) were poured.
20. An electrophoresis apparatus was assembled.
21. 20 µg of protein was loaded into each well of the gel.
22. Electrophoretic separation was carried out under conditions of 25 mA, 270 V, const. A, until the samples spread out on the gel.
19. During the separation, membranes were activated by immersing them in methanol for 10 minutes, and then by incubating them in transfer buffer for 10 minutes.
20. After the electrophoresis was completed, the apparatus was disassembled, Western Blot sandwiches were assembled, placed in a cassette, and transfer was carried out for 75 minutes, 270 V, 270 mA, const. V.
21. The device was disassembled, the membranes were rinsed in TBS-T buffer for 5 minutes at RT with shaking.
22. Then the membranes were placed in 5% skimmed milk or Phospho blocker for 1 hour at RT with rocking.
19. Membranes were washed 3 times for 10 minutes in TBS-T buffer.
20. Primary antibodies diluted in TBS-T buffer were added (mouse anti-ERK 1/2 (C9) sc-7383, Santa Cruz, 1:500; mouse anti-p-ERK (E-4) sc-7838, Santa Cruz, 1:500; rabbit anti- Akt, 9272S, Cell Signalling, 1:500; rabbit anti-pAkt (S473) (D9E), 4060L, Cell Signalling, 1:500; mouse anti-Actin, clone C4, MAB1501, Merck, 1:4000), and left overnight at +4°C.

### Day 6

29. The next day, the membranes were brought to room temperature, rinsed 3 times for 10 minutes with TBS-T buffer.
30. Secondary antibodies diluted in TBS-T buffer were added (goat anti-rabbit IgG-HRP, sc-2004, Santa Cruz, 1:4000; m-IgGκB- HRP (mouse), sc-516102, Santa Cruz, 1:4000), and incubated for 2 hours at room temperature with shaking.
31. The membranes were rinsed 3 times for 10 minutes with TBS-T buffer.
32. Visualized using the Amersham ECL Western Blotting Detection reagent.

**Results and conclusions:** The molecule labeled as PER154A and PER160A blocks the basic downstream signal transduction pathways in relation to RASG12V in cells with the RASG12V mutation (Illustration 7).

### Example 3.

**Analysis of the ability of selected molecules to restore the GTPase activity of the mutated form of KRASG12V protein based on the GTPase test.**

The example illustrates the restoration of GTPase activity in the case of the mutated form of the protein KRASG12V after the application of selected molecules PER154A, PER170A and PER176A with therapeutic potential.

GTP-ases act as molecular switches between the active state (bound to GTP) and the inactive state (bound to GDP) and are an essential element of signal transduction pathways. The process of protein switching between active and inactive forms is supported by two families of proteins: GTPase-activating proteins (GAP) and guanine nucleotide exchange factors (GEF). The GTPase-Glo test allows for the assessment of the activity of GTP-ases, GAP proteins, and GEF proteins by detecting the remaining GTP after hydrolysis reaction. The unhydrolyzed GTP is then converted to ATP with the help of GTPase-Glo Reagent. The ATP is then used as a substrate for the oxidation reaction of luciferin by luciferase, resulting in bioluminescence. The intensity of luminescence is directly proportional to the amount of ATP. Therefore, the activity of proteins is inversely proportional to the emitted luminescence. The test enables measurement of the internal activity of GTP-ases, the activity stimulated by GAP proteins, and the activity of GAP and GEF proteins.
The general rule of the test: 1:1:2
2xGTP solution + GTPase + GAP + molecule: GTPase Glo Reagent: Detection Reagent

### Protocol:

1. 500 ng of KRAS protein (WT or G12V) and 500 ng of RASA1 protein were added to each well of a 96-well plate.
2. Then, test compounds were added at specified concentrations.
3. The total volume was brought up to 25 µl with a reaction mixture containing 10 µM GTP, 1 µM DTT, and GTPase/GAP Buffer.
4. The plate was incubated for 3.5 hours at room temperature on a shaker.
5. 25 µl of a reaction mixture containing GTPase-Glo Reagent 500X, 1 mM ADP, and GTPase-Glo Buffer was added to each well.
6. The plate was then incubated for 30 minutes at room temperature with shaking.
7. 50 µl of Detection Reagent was added to each well.
8. The plate was incubated for 8 minutes with shaking at room temperature.
9. Luminescence was measured using a plate reader.

**Results and conclusions:** Molecules labeled as PER154A, PER160A, and PER176A show the ability to partially restore GTPase activity in the case of the mutated form of the KRASG12V protein (Illustration 8, Illustration 9, Illustration 10).

### Example 4

### EVALUATION OF IC50 VALUES FOR SELECTED MOLECULES WITH THERAPEUTIC POTENTIAL

IC₅₀ (half-maximal inhibitory concentration) is the concentration value of a tested compound which inhibits biochemical and biological functions of cells in half of the tested population. Determining this indicator is extremely important for assessing the cytotoxicity of molecules with therapeutic potential.

The first step in developing the methodology for determining IC₅₀ was to conduct density tests to determine the appropriate number of cells to use in cytotoxicity tests. The different content of bovine fetal serum in the culture medium was also investigated to rule out any potential impact on the reduction in cell count, regardless of the analyzed molecules.

As a research model, colon cancer and lung cancer cell lines (sensitive lines) were used, including SW620, SW480, and COR-L23, respectively, which are characterized by the KRASG12V gene mutation and lack of other alterations in genes belonging to the RAS protein signaling pathway, as well as a stable line of normal cells (mouse fibroblasts), which serve as a platform for evaluating the impact of tested compounds on non-cancer cells. Below is the result for one of the analyzed cell lines with the mutation. Similar differences in IC50 values compared to normal cells were obtained for the other cell lines mentioned above with the RASG12V mutation.

The effect of molecules on the biology of selected cell lines to assess their cytotoxicity using the IC50 indicator was carried out according to the protocol below:
1. The cells of the tested lines were seeded in wells of a 96-well plate at specified densities.
2. After seeding, the cells were subjected to a 24-hour incubation in medium with a 1% and 2.5% addition of serum (sensitive lines) or in medium with a 0.5% addition of serum (normal line).
3. The cells were exposed to selected molecules at selected concentrations. The control was provided by the culture medium without any added compounds.
   Concentrations of the tested molecules [uM]:
   □ NIH3T3: 500; 250; 125; 62,5; 31,25
   □ SW620: 60; 30; 15; 7,5; 3,75
4. After 72 hours of incubation, the culture medium was removed and the cells were washed twice with buffer saline solution (PBS). Then, the cells were incubated for 10 minutes with a 0.1% solution of crystal violet at room temperature (Merck Millipore).
5. After 10 minutes, the crystal violet solution was removed and the wells of the 96-well plate were washed twice with PBS solution.
4. 50% ice-cold acetic acid solution was added to each well and incubated again for 10 minutes at room temperature on a shaker at 650 rpm.
5. Absorbance measurement was performed using a plate reader, at a wavelength of λ = 540 nm

**Results and conclusions:** The PER154A molecule exhibits about 85 times greater and the PER160A molecule over 36 times greater toxicity towards KRASG12V positive cells (SW620) compared to normal cells (NIH/3T3) (Illustration 11).

### Example 5. ACUTE TOXICITY AFTER ORAL ADMINISTRATION (VIA THE ORAL ROUTE) (ACUTE TOXICITY TEST TG425)

The study was planned according to Acute Toxicity Test 425 (oral administration) - higher-lower-dose procedure, in accordance with the OECD regulation of October 3, 2008. The test procedure described in these guidelines is useful in minimizing the number of animals needed to estimate the acute toxicity after oral administration of potential drugs. In addition to the LD50 assessment and confidence intervals, the test allows for the observation of signs of toxicity. For this purpose, an experiment was agreed upon with the Local Ethics Committee for Animal Experimentation, using BALB/c mice. The TG425 method allows for the estimation of LD50 values with confidence intervals, and the results allow for the classification of substances according to the global harmonized system for the classification of chemicals that cause acute toxicity.

A sequential test, utilizing a maximum of 5 animals, was applied, which is known as the limit test. A test dose of either 2000 or uniquely 5000 mg/kg can be used. The testing procedures at 2000 and 5000 mg/kg differ slightly (see Sections 23-25 for the limit test at 2000 mg/kg and Sections 26-30 for the limit test at 5000 mg/kg). The selection of a sequential testing plan increases the statistical power, and a deliberate deviation from the limit test rejection procedure was made for compounds with an LD50 near the limit dose; i.e. committing an error on the side of caution. As with any limit test protocol, the probability of correct compound classification will decrease, as the actual LD50 value more closely resembles the limit dose.

### Experiment process:

1. Female Balb/c mice, which were 50-56 days postpartum, were used for the purposes of this experiment.
2. Mice underwent a 1-week quarantine after being transported to the laboratory.
3. The studied molecules (PER154A, PER160A, PER170A, PER176A) were suspended according to the TG425 protocol, i.e. 40 mg of substance in 200 ml of laboratory corn oil and orally administered through a plastic gastro probe (used: 20ga (0.9 mm OD x 0.5 mm ID), 38 mm), which was suitable for the size of the animal (17-21 g). The mice were observed and weighed almost daily.
4. Mouse housing conditions: up to 4 mice per cage; temperature: 23.2 - 23.40°C; humidity: 27.6 - 40.9% relative humidity.
5. Two weeks later (time counted separately for each mouse), a resection was performed to analyze internal organs for any abnormalities.

### Results and conclusions:

The PER154A compound turned out to be safe for the subsequent mice, which survived without any visual signs of toxicity. Minor fluctuations in body weight were not associated with any observed changes in behavior, so this may be typical for this strain of mice (the effect of stress, food, or another factor). The appearance of the internal organs of the tested mice and the control mice after euthanasia - no visual signs of toxicity (Illustration 12 and Illustration 13).

### Literature

1. COSMIC. baza danych. protokó 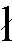 dost 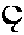 pu: http://cancer.sanger.ac.uk/cosmic
2. Stewart BW and Wild CP. World Cancer Report 2014. International Agency for Research on Cancer, World Health Organization
3. Lu S, Jang H, Nussinov R, Zhang J. The Structural Basis of Oncogenic Mutations G12, G13 and Q61 in Small GTPase KRAS4B. Sci Rep. 2016; 6: 21949
4. Khrenova MG, Mironov VA, Grigorenko BL, Nemukhin AV. Modeling the role of G12V and G13V RAS mutations in the RAS-GAP-catalyzed hydrolysis reaction of guanosine triphosphate. Biochemistry. 2014; 53(45): 7093-9
5. Khrenova MG, Grigorenko BL, Mironov VA, Nemukhin AV. Why does mutation of Gln61 in RAS by the nitro analog NGln maintain activity of RAS-GAP in hydrolysis of guanosine triphosphate? Proteins. 2015; 83(11): 2091-9
6. Scheffzek K, Ahmadian MR, Kabsch W, Wiesmüller L, Lautwein A, Schmitz F, Wittinghofer A. The Ras-RasGAP Complex: Structural Basis for GTPase Activation and Its Loss in Oncogenic Ras Mutants Science. 1997; 277(5324): 333-8
7. Prior A, Lewis PD, Mattos C. A comprehensive survey of Ras mutations in cancer Cancer Res. 2012; 72(10): 2457-67

## Claims

1. A chemical compound described by formula (I) known as
6-phenyl- 5,6,6a,7,8,9,10,10a-octahydro-7,10-methanophenanthrydin-2-carboxyamide (IUPAC: 10-phenyl-9-azatetracyclo[10.2.1.0^2,11.0^3,8]pentadeca-3(8),4,6-triene-5- carboxamide) its derivatives, and a pharmaceutical composition containing thiscompound as an active substance used in antitumor therapy in tumors whose cells exhibit the expression of the RAS protein with a mutation causinga change from glycine 12 to valine (codon 12 glycine-valine; G12V). 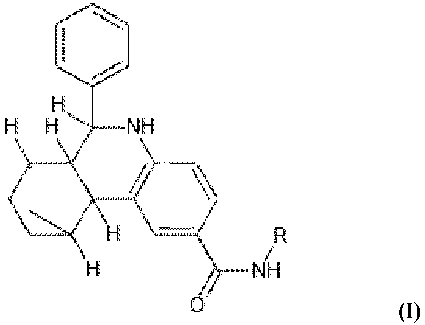 where R is a group: 2H-1,3-benzodioxole; 2,3-dihydro-1,4-benzodioxane; cyclopentyl; 2- chlorophenyl; 4-chlorophenyl; 4-fluorophenyl; 4-methoxyphenyl; 4-(2-propyl)phenyl; 3,4- difluorophenyl; 3-chloro-4-fluorophenyl; 4-fluorophenyl; 3,4-dimethylphenyl; 3,4- dimethoxyphenyl; 4-bromophenyl; 3,4-difluorophenyl; 3-chloro-4-methylphenyl; 4-ethylphenyl; 4-bromo-3-chlorophenyl.

2. The chemical compound described by formula (I) and a pharmaceutical composition containing this compound as an active substance for use in the treatment, according to claim 1, **characterized in that** the patient's cancer cells express RASG12V protein, where RASG12V denotes the protein encoded by the RAS genes, in which Glycine (G) encoded by codon 12 is converted to Valine (V).

3. The chemical compound described by formula (I) and a pharmaceutical composition containing this compound as an active substance for use in the treatment, according to claim 1, are **characterized in that**, as a result of restoring GTP-ase activity, due to their action, the oncogenic activity of RASG12V proteins is blocked.

4. The chemical compound described by formula (I) and a pharmaceutical composition containing this compound as an active substance for use in the treatment, a according to claim 1, **characterized in that** they restore the GTPase activity of RASG12V proteins.

5. The chemical compound described by formula (I) and a pharmaceutical composition containing this compound as the active substance for use in treatment are, according to claim 1, **characterized in that** they block the RASG12V protein-dependent signal in the cell.

6. The chemical compound described by formula (I) and a pharmaceutical composition containing this compound as the active substance for use in treatment are, according to claim 1, **characterized by** the effect of their action involving the contact of the cell with the described compound in the claim 1.

7. The chemical compound described by formula (I) and the pharmaceutical composition containing this compound as the active substance for use in treatment, according to claim 1, **characterized in that** the cell on which it acts is a human cancer cell and/or a cancer stem cell and/or is a colorectal cancer cell and/or cancer cells of pancreatic, colorectal, bladder, lung, ovarian, melanoma, and acute myeloid leukemia and/or any cancer cell with the mutation RAS^{G12V}.

8. The chemical compound described by formula (I) and a pharmaceutical composition containing this compound as the active substance for use in the treatment are according to claim 1, significant in that the cell they act on is a pancreatic cancer cell, colorectal cancer, bladder cancer, lung cancer, ovarian cancer, melanoma, and acute myeloid leukemia expressing the RAS^{G12V} protein, where RAS^{G12V} signifies the protein encoded by the RAS genes, in this protein, Glycine (G) encoded by codon 12 is replaced by Valine (V).

9. The chemical compound described by formula (I) and a pharmaceutical composition containing this compound as an active substance for use in treatment are, according to claim 1, **characterized by** their ability to change the conformation of the side chain and RAS skeleton fragment in the part containing the G12V mutation. This enables correct positioning of Q61, previously impossible due to steric hindrance in the form of the V12 side chain, or the ability to replace NE2 Q61 with a nitrogen atom.

10. The chemical compound described by formula (I) and a pharmaceutical composition containing this compound as the active substance for use in treatment are, according to claim 1, **characterized in that** the pharmaceutical composition optionally contains permissible carriers, solvents, preservatives, and excipients, preferably the carrier in the pharmaceutical composition is a liquid from a group containing water, alcohol, liquid glucose, or aqueous solution, DMSO.

11. The chemical compound described by formula (I) and the pharmaceutical composition containing this compound as an active substance for use in treatment are, according to claim 1, significant in that the effective concentration in tissues restores the activity of the mutated RASG12V protein by at least 5%, in particular at least 10% to 90%, especially 15-50%.

12. The chemical compound described by formula (I) and the pharmaceutical composition containing this compound as an active substance for use in treatment are, according to claim 1, **characterized in that** the pharmaceutical composition contains at least one active substance from the group, including the chemical compound described by formula
(I) and its derivatives.

13. The chemical compound described by formula (I) and a pharmaceutical composition containing this compound as the active substance for use in the treatment are, according to claim 1, **characterized in that** the pharmaceutical composition contains a non- toxic concentration of the active substance for restoring the GTP-ase activity of RASG12V proteins in human cancer cells **characterized by** this mutation.

14. The chemical compound described by formula (I) is significant in that it restores the GTPase activity of RASG12V proteins in in vitro and in vivo studies as well as in functional tests.

15. The chemical compound described by formula (I) is intended for use in restoring at least partial GTPase activity of the RASG12V protein.
